# EUROPEAN PATENT SPECIFICATION

(11) **EP 3 573 942 B1**
(45) Date of publication and mention of the grant of the patent: **01.12.2021**
(21) Application number: 17823004.1
(22) Date of filing: 15.12.2017
(51) Int. Cl.: C07C 6/12

(54) **TRANSALKYLATION PROCESS AND CATALYST COMPOSITION USED THEREIN**
TRANSALKYLIERUNGSVERFAHREN UND DARIN VERWENDETE KATALYSATORZUSAMMENSETZUNG
PROCÉDÉ DE TRANSALKYLATION ET COMPOSITION DE CATALYSEUR UTILISÉ DANS CELUI-CI

(30) Priority: 25.01.2017 US 201762450122 P; 20.03.2017 EP 17161741
(43) Date of publication of application: 04.12.2019
(73) Proprietor: ExxonMobil Chemical Patents Inc., Baytown, TX 77520 (US)
(72) Inventor: LOVELESS, Brett, T., Houston, TX 77044 (US); BEECKMAN, Jean, W., Columbia, MD 21044 (US); OLIVERI, Christopher, G., Humble, TX 77396 (US); WEIGEL, Scott, J., Allentown, PA 18104 (US); IDE, Matthew, S., Doylestown, PA 18902 (US); BAUMRUCKER, Theresa, A., Spring, TX 77386 (US)
(74) Representative: ExxonMobil Chemical Europe Inc.
(86) International application number: PCT/US2017/066633
(87) International publication number: WO 2018/140149

(56) References cited:
- WO-A1-2007/139629
- WO-A1-2008/100658
- WO-A1-2014/182294
- US-A1- 2008 171 649
- US-A1- 2008 171 901
- US-A1- 2008 171 902

## Description

### FIELD OF THE INVENTION

The present invention relates to a process for the transalkylation of aromatics, particularly the transalkylation of poly-isopropylbenzene (PIPB) with benzene to produce cumene and the transalkylation of poly-ethylbenzene (PEB) with benzene to produce ethylbenzene.

### BACKGROUND OF THE INVENTION

Ethylbenzene is a valuable commodity chemical and is used in the production of styrene monomer. Cumene (isopropylbenzene) is also a valuable commodity chemical and is used in the production of phenol and acetone.

Presently, ethylbenzene is often produced by a liquid phase alkylation process from benzene and ethylene in the presence of an alkylation catalyst. The liquid phase process operates at a lower temperature than its vapor phase counterpart. One advantage of the liquid phase alkylation is a lower yield of undesired by-products, poly-alkylated aromatic compound(s). The alkylation of aromatic hydrocarbon compounds employing zeolite catalysts is known and understood in the art. U.S. Pat. No. 5,334,795 describes the liquid phase alkylation of benzene with ethylene in the presence of MCM-22 to produce ethylbenzene; and U.S. Pat. No. 4,891,458 discloses liquid phase alkylation and transalkylation processes using zeolite beta.

Cumene is often produced by a liquid phase alkylation process from benzene and propylene in the presence of a zeolite-based alkylation catalyst. U.S. Pat. No. 4,992,606 discloses a process for preparing cumene using MCM-22 in liquid phase.

Commercial alkylation processes for the production of ethylbenzene and cumene typically produce certain poly-alkylated by-products in addition to desired ethylbenzene and cumene. The poly-alkylated aromatic compound(s) may be transalkylated with benzene or other alkylatable aromatic compound(s) to produce additional ethylbenzene or cumene. This transalkylation reaction may be accomplished by feeding the poly-alkylated aromatic compound(s) through a transalkylation reactor operated under suitable conditions and in the presence of a transalkylation catalyst. U.S. Pat. No. 5,557,024 discloses a process for preparing short chain alkyl aromatic compounds using MCM-56 and the use of zeolite catalysts such as MCM-22, zeolite X, zeolite Y and zeolite beta for the transalkylation of the poly-alkylated aromatic compound(s). US2008/171902, US2008/171901 and US2008/171649 disclose further transalkylation processes for the preparation of ethylbenzene and/or cumene.

Despite the advances in the liquid phase aromatic alkylation process, there is a need for an improved transalkylation process which has a higher conversion of the poly-alkylated aromatic compounds to the desired mono-alkylated aromatic compound, such as ethylbenzene or cumene.

### SUMMARY OF THE INVENTION

Higher conversion of the poly-alkylated aromatic compounds to the desired mono-alkylated aromatic compounds in transalkylation processes may be achieved by the use of a higher-activity transalkylation catalyst composition. It has been discovered that a higher-activity transalkylation catalyst composition may be produced by increasing the external surface area/volume (SA/V) ratio of the transalkylation catalyst composition to a selected range of 40 cm⁻¹ to 85 cm⁻¹ combined with reducing the silica-to-alumina (Si/Ah) molar ratio of the zeolite in the composition to a range of 10 to 15.

In one aspect, the invention is a process for producing ethylbenzene or cumene comprising one or more steps as defined in the appended claims. In step (a), a transalkylation catalyst composition, described below, is provided to a reaction zone. In step (b), a stream comprising poly-alkylated benzene and an alkylatable aromatic compound stream comprising benzene are provided to the reaction zone. The poly-alkylated benzene stream comprises di-ethylbenzene or di-isopropylbenzene. In step (c), the poly-alkylated benzene stream is contacted with the benzene stream in the presence of the aforementioned transalkylation catalyst composition under at least partial liquid phase transalkylation conditions to produce a transalkylation effluent stream. Such effluent comprises ethylbenzene or cumene. The liquid phase transalkylation conditions include a temperature of 100°C to 300°C and a pressure of 200 kPa-a to 6000 kPa-a.

In one or more embodiments of the process, the catalytic activity of the transalkylation catalyst composition of this invention is higher (i.e., lower molar silica-content) than the catalytic activity of a lower-activity (i.e., higher molar silica-content) transalkylation catalyst composition which comprises said zeolite and has a silica-alumina molar ratio in the range of 25 to 37 when the catalysts are compared under equivalent transalkylation conditions.

In one or more embodiments of the process, the higher-activity (i.e., lower silica-content) transalkylation catalyst composition of this invention when employed in a process for producing ethylbenzene or cumene, exhibits a weight hourly space velocity of the poly-alkylated benzene stream that is higher than the weight hourly space velocity of a lower-activity (i.e., higher silica-content) transalkylation catalyst composition employed in such process, where the catalysts are compared under equivalent transalkylation conditions. In another embodiment, a portion of the stream comprising benzene is contacted with an alkylating agent stream under alkylation conditions and in the presence of an alkylation catalyst to produce an alkylation effluent which comprises a mono-alkylated benzene and the poly-alkylated benzene. Thereafter, the alkylation effluent is separated to recover the poly-alkylated benzene stream in which a portion is supplied to step (b) of the process for producing ethylbenzene or cumene.

In still another embodiment, the benzene stream is an impure stream which further comprises nitrogenous impurities. The impure stream is contacted with a treatment material under treatment conditions to remove at least a portion of the nitrogenous impurities. The treatment material is selected from the group consisting of clay, resin, activated alumina, a molecular sieve and combinations thereof.

### DETAILED DESCRIPTION OF THE INVENTION

Improved catalytic activity is exhibited by the transalkylation catalyst composition of this invention, described herein, when used in a process for producing a mono-alkylated aromatic compound, preferably ethylbenzene or cumene, by the transalkylation of a poly-alkylated aromatic compound with an alkylatable aromatic compound, preferably benzene, in the presence of such composition under at least partial liquid phase transalkylation conditions.

To achieve the improved catalytic activity, the external surface area/volume ratio of transalkylation catalyst composition is increased to a selected range of 40 cm⁻¹ to 85 cm⁻¹, and the silica-to-alumina (Si/Ah) molar ratio of the zeolite is reduced to a selected range of 10 to 15. The zeolite has a framework structure selected from the group consisting of FAU, BEA^{∗}, MOR, MWW and mixtures thereof.

### Definitions

The term "alkylatable aromatic compound" as used herein means an aromatic compound that may receive an alkyl group. One non-limiting example of an alkylatable aromatic compound is benzene.

The term "alkylating agent" as used herein means a compound which may donate an alkyl group to an alkylatable aromatic compound. Non-limiting examples of an alkylating agent are ethylene, propylene, and butylene. Another non-limiting example is any poly alkylated aromatic compound that is capable of donating an alkyl group to an alkylatable aromatic compound.

The term "aromatic" as used herein in reference to the alkylatable aromatic compounds which are useful herein is to be understood in accordance with its art-recognized scope which includes substituted and unsubstituted mono- and polynuclear compounds. Compounds of an aromatic character which possess a heteroatom (e.g., N or S) are also useful provided they do not act as catalyst poisons, as defined below, under the reaction conditions selected.

The term "at least partial liquid phase" as used herein, means a mixture having at least 1 wt.% liquid phase, optionally at least 5 wt.% liquid phase, at a given temperature, pressure, and composition.

The term "framework type" as used herein has the meaning described in the "Atlas of Zeolite Framework Types," by Ch. Baerlocher, W.M. Meier and D.H. Olson (Elsevier, 5th Ed., 2001).

The term "MCM-22 family material" (or "MCM-22 family molecular sieve"), as used herein, can include:
(i) molecular sieves made from a common first degree crystalline building block "unit cell having the MWW framework topology." A unit cell is a spatial arrangement of atoms which is tiled in three-dimensional space to describe the crystal as described in the "Atlas of Zeolite Framework Types," by Ch. Baerlocher, W.M. Meier and D.H. Olson (Elsevier, 5th Ed., 2001);
(ii) molecular sieves made from a common second degree building block, a 2-dimensional tiling of such MWW framework type unit cells, forming a "monolayer of one unit cell thickness," preferably one c-unit cell thickness;
(iii) molecular sieves made from common second degree building blocks, "layers of one or more than one unit cell thickness", wherein the layer of more than one unit cell thickness is made from stacking, packing, or binding at least two monolayers of one unit cell thick of unit cells having the MWW framework topology. The stacking of such second degree building blocks can be in a regular fashion, an irregular fashion, a random fashion, and any combination thereof; or
(iv) molecular sieves made by any regular or random 2-dimensional or 3- dimensional combination of unit cells having the MWW framework topology.

The MCM-22 family materials are characterized by having an X-ray diffraction pattern including d-spacing maxima at 12.4±0.25, 3.57±0.07 and 3.42±0.07 Angstroms (either calcined or as-synthesized). The MCM-22 family materials may also be characterized by having an X-ray diffraction pattern including d-spacing maxima at 12.4±0.25, 6.9±0.15, 3.57±0.07 and 3.42±0.07 Angstroms (either calcined or as-synthesized). The X-ray diffraction data used to characterize the molecular sieve are obtained by standard techniques using the K-alpha doublet of copper as the incident radiation and a diffractometer equipped with a scintillation counter and associated computer as the collection system.

Members of the MCM-22 family include, but are not limited to, MCM-22 (described in U.S. Patent No. 4,954,325), PSH-3 (described in U.S. Patent No. 4,439,409), SSZ-25 (described in U.S. Patent No. 4,826,667), ERB-1 (described in European Patent 0293032), ITQ-1 (described in U.S. Patent No. 6,077,498), ITQ-2 (described in International Patent Publication No. WO97/17290), ITQ-30 (described in International Patent Publication No. WO2005118476), MCM-36 (described in U.S. Patent No. 5,250,277), MCM-49 (described in U.S. Patent No. 5,236,575), MCM-56 (described in U.S. Patent No. 5,362,697; and an EMM-10 family molecular sieve (described or characterized in U.S. Patent Nos. 7,959,899 and 8,110,176; and U.S. Patent Application Publication No. 2008/0045768), such as EMM-10, EMM-10-P, EMM-12 and EMM-13.

Related zeolites to be included in the MCM-22 family are UZM-8 (described in U.S. Patent No. 6,756,030) and UZM-8HS (described in U.S. Patent No. 7,713,513), UZM-37 (described in U.S. Patent No. 8,158,105), all of which are also suitable for use as the molecular sieve of the MCM-22 family. Typically, the molecular sieve of the MCM-22 family is in the hydrogen form and having hydrogen ions, for example, acidic.

Typically, the molecular sieve of the MCM-22 family is in the hydrogen form and having hydrogen ions, for example, acidic.

The term "mono-alkylated aromatic compound" means an aromatic compound that has only one alkyl substituent. Non-limiting examples of mono-alkylated aromatic compounds are ethylbenzene, iso-propylbenzene (cumene) and sec-butylbenzene.

The term "poly-alkylated aromatic compound" as used herein means an aromatic compound that has more than one alkyl substituent. A non-limiting example of a poly-alkylated aromatic compound is poly-ethylbenzene, e.g., di-ethylbenzene, tri-ethylbenzene, and poly-isopropylbenzene, e.g., di-isopropylbenzene, and tri-isopropylbenzene.

The term "regenerated" when used in connection with the alkylation catalyst or the transalkylation catalyst herein means an at least partially deactivated catalyst that has been treated under controlled conditions of oxygen content and temperature to remove at least a portion of the coke deposited or to remove at least a portion of adsorbed catalyst poisons and thereby increase the catalytic activity of such material or catalyst.

The term "fresh" when used in connection with the molecular sieve, the guard bed material, the alkylation catalyst, or the transalkylation catalyst herein means the molecular sieve or such catalyst has not been used in a catalytic reaction after being manufactured.

The term "impurities" as used herein includes, but is not limited to, compounds having at least one of the following elements: nitrogen, halogens, oxygen, sulfur, arsenic, selenium, tellurium, phosphorus, and Group 1 through Group 12 metals.

### Process

In one aspect, the invention is a process for producing ethylbenzene or cumene, comprising one or more steps. In step (a) of the process, a transalkylation catalyst composition, described herein, is provided to a reaction zone. In step (b) of the process, a stream comprising poly-alkylated benzene and an alkylatable aromatic compound stream comprising benzene are provided to the reaction zone. The poly-alkylated benzene stream used to produce ethylbenzene comprises di-ethylbenzene. The poly-alkylated benzene stream used to produce cumene is di-isopropylbenzene. In step (c) of the process, the poly-alkylated benzene stream is contacted with the benzene stream in the presence of the aforementioned transalkylation catalyst composition under at least partial liquid phase transalkylation conditions to produce a transalkylation effluent stream comprising ethylbenzene or cumene.

The products of the transalkylation reaction of the invention include ethylbenzene from the transalkylation reaction of a poly-ethylbenzene, such as di-ethylbenzene, with benzene, or cumene from the transalkylation reaction of poly-isopropylbenzene, such as di-isopropylbenzene, with benzene.

The transalkylation effluent is separated in a conventional separation system to recover the desired ethylbenzene stream or a cumene stream. Such conventional separation system, includes, for example, a benzene column, an ethylbenzene or cumene column, and a poly-alkylated column to recover the poly-ethylbenzene stream or the poly-isopropylbenzene stream.

The poly-alkylated benzene stream is produced from an alkylation process step which is particularly intended to produce mono-alkylated aromatic compounds, such as ethylbenzene and cumene in an alkylation step; however, the alkylation step will normally produce some poly-alkylated aromatic compounds, such as poly-ethylbenzene or poly-isopropylbenzene.

In an alkylation step, a portion of the stream comprising benzene, or a portion thereof, is contacted with a stream comprising an alkylating agent under alkylation conditions and in the presence of an alkylation catalyst to produce an alkylation effluent. This effluent stream comprises mono-alkylated benzene and poly-alkylated benzene stream. Preferably, the alkylating agent is ethylene and used to alkylate benzene to produce ethylbenzene, or propylene and used to alkylate benzene to produce cumene. In one embodiment, the mono-alkylated benzene is ethylbenzene and said poly-alkylated benzene is poly-ethylbenzene. In another embodiment, the mono-alkylated benzene is cumene and said poly-alkylated benzene is poly-isopropylbenzene.

The alkylation effluent is separated to recover said poly-alkylated benzene stream. The recovered poly-alkylated benzene stream is then be supplied to step (b) of the process to produce ethylbenzene or cumene.

In one or more embodiments, the stream comprising benzene is an impure stream which further comprises nitrogenous impurities. The process of this invention, may further comprise the step of contacting the impure stream with a treatment material under treatment conditions to remove at least a portion of the nitrogenous impurities. The treatment material is selected from the group consisting of clay, resin, activated alumina, Linde type X, Linde type A and combinations thereof.

When a treatment material is used to remove a portion of impurities, suitable treatment conditions include a temperature from 30°C to 200°C, and preferably between 60°C to 150°C, a weight hourly space velocity (WHSV) of from 0.1 hr⁻¹ and 200 hr⁻¹, preferably from 0.5 hr⁻¹ to 100 hr⁻¹, and more preferably from 1.0 hr⁻¹ to 50 hr⁻¹; and a pressure between ambient and 3000 kPa.

### Transalkylation and Alkylation Conditions

The process for producing ethylbenzene or cumene of this invention is conducted such that the organic reactants, i.e., the alkylatable aromatic compound, e.g. the benzene, and the alkylating agent, i.e. poly-alkylated benzene or ethylene or propylene, are brought into contact with an alkylation catalyst or a transalkylation catalyst. The contact is made in a suitable reaction zone such as, for example, in a flow reactor containing a fixed bed of the catalyst composition, under effective alkylation or transalkylation conditions. Such conditions include at least partial liquid phase transalkylation conditions or at least partial liquid phase alkylation conditions.

In one or more embodiments, the reactants can be neat, i.e., free from intentional admixture or dilution with other material, or they can include carrier gases or diluents such as, for example, hydrogen or nitrogen.

The at least partial liquid phase conditions for transalkylation can include at least one of the following: a temperature of 100°C to 300°C, or from 150°C to 260°C, a pressure of 200 kPa to 6000 kPa, or 200kPa to 500 kPa, a weight hourly space velocity (WHSV) based on the total feed of 0.5 hr⁻¹ to 100 hr⁻¹ on total feed, and aromatic/poly-alkylated aromatic compound weight ratio 1:1 to 6:1.

When the poly-alkylated aromatic compounds are poly-ethylbenzenes and are reacted with benzene to produce ethylbenzene, the transalkylation conditions include a temperature of from 220°C to 260°C, a pressure of from 300 kPa to 400 kPa, weight hourly space velocity of 2 to 6 on total feed and benzene/PEB weight ratio 2:1 to 6:1.

When the poly-alkylated aromatic compounds are poly-isopropylbenzenes (PIPBs) and are reacted with benzene to produce cumene, the conditions for transalkylation include a temperature of from 100°C to 200°C, a pressure of from 300 kPa to 400 kPa, a weight hourly space velocity of 1 to 10 on total feed and benzene/PIPB weight ratio 1:1 to 6:1.

The at least partial liquid phase conditions for alkylation can include at least one of the following: a temperature of from 10°C and 400°C, or from 10°C to about 200°C, or from 150°C to about 300°C, a pressure up to 25000 kPa, or up to 20000 kPa, or from 100 kPa to 7000 kPa, or from 689 kPa to 4601 kPa, a molar ratio of alkylatable aromatic compound to alkylating agent of from 0.1:1 to 50:1, preferably from 0.5:1 to 10:1, and a feed weight hourly space velocity (WHSV) of between 0.1 and 100 hr⁻¹, or from 0.5 to 50 hr⁻¹, or from 10 hr⁻¹ to 100 hr⁻¹.

When benzene is alkylated with ethylene to produce ethylbenzene, the alkylation reaction may be carried out under at least partially liquid phase conditions for alkylation which include a temperature between 150°C and 300°C, or between 200°C and 260°C, a pressure up to 20000 kPa, preferably from 200 kPa to 5600 kPa, a WHSV of from 0.1 hr⁻¹ to 50 hr⁻¹, or from 1 hr⁻¹ and 10 hr⁻¹ based on the ethylene feed, and a ratio of the benzene to the ethylene in the alkylation reactor from 1:1 to 30:1 molar, preferably from 1:1 to 10:1 molar.

When benzene is alkylated with propylene to produce cumene, the reaction may be carried out under at least partially liquid phase conditions for alkylation which include a temperature of up to 250°C, preferably from 10°C to 200°C; a pressure up to 25000 kPa, preferably from 100 kPa to 3000 kPa; and a WHSV of from 1 hr⁻¹ to 250 hr⁻¹, preferably from 5 hr⁻¹ to 50 hr⁻¹, preferably from 5 hr⁻¹ to 10 hr⁻¹ based on the ethylene feed.

### Alkylatable Aromatic Compounds

Substituted alkylatable aromatic compounds which can be alkylated must possess at least one hydrogen atom directly bonded to the aromatic nucleus. The aromatic rings can be substituted with one or more alkyl, aryl, alkaryl, alkoxy, aryloxy, cycloalkyl, halide, and/or other groups which do not interfere with the alkylation reaction.

Suitable alkylatable aromatic hydrocarbons include benzene, naphthalene, anthracene, naphthacene, perylene, coronene, and phenanthrene, with benzene being preferred in the present invention.

Generally the alkyl groups, which can be present as substituents on the aromatic compound, contain from 1 to 22 carbon atoms and usually from 1 to 8 carbon atoms, and most usually from 1 to 4 carbon atoms.

Suitable alkyl substituted aromatic compounds include toluene (also preferred), xylene, isopropylbenzene, normal propylbenzene, alpha-methylnaphthalene, ethylbenzene, cumene, mesitylene, durene, p-cymene, butylbenzene, pseudocumene, o-diethylbenzene, m-diethylbenzene, p-diethylbenzene, isoamylbenzene, isohexylbenzene, pentaethylbenzene, pentamethylbenzene; 1,2,3,4-tetraethylbenzene; 1,2,3,5-tetramethylbenzene; 1,2,4-triethylbenzene; 1,2,3-trimethylbenzene, m-butyltoluene; p-butyltoluene; 3,5-diethyltoluene; o-ethyltoluene; p-ethyltoluene; m-propyltoluene; 4-ethyl-m-xylene; dimethylnaphthalene; ethylnaphthalene; 2,3-dimethyl anthracene; 9-ethylanthracene; 2-methylanthracene; o-methylanthracene; 9,10-dimethylphenanthrene; and 3-methyl-phenanthrene. Higher molecular weight alkylated aromatic hydrocarbons can also be used as starting materials and include aromatic hydrocarbons such as are produced by the alkylation of aromatic hydrocarbons with olefin oligomers. Such products are frequently referred to in the art as alkylate and include hexylbenzene, nonylbenzene, dodecylbenzene, pentadecylbenzene, hexyltoluene, nonyltoluene, dodecyltoluene, pentadecyltoluene, etc. Very often, alkylate is obtained as a high boiling fraction in which the alkyl group attached to the aromatic nucleus varies in size from C₆ to C₁₂. When cumene or ethylbenzene is the desired product, the present process produces acceptably little by-products such as xylenes. The xylenes made in such instances may be less than 500 ppm.

Reformate containing substantial quantities of benzene, toluene and/or xylene constitutes a useful feed for the process of this invention.

### Alkylating Agents

The alkylating agents, which are useful in one or more embodiments of this invention, generally include any aliphatic or aromatic organic compound having one or more available alkylating olefinic groups capable of reaction with the alkylatable aromatic compound. Preferably, the alkylating agent comprises an olefinic group having from 1 to 5 carbon atoms, or a poly-alkylated aromatics compound(s). More preferably, the alkylation agents are poly-ethylbenzene and poly-isopropylbenzene for the transalkylation reaction, and ethylene and propylene for the alkylation reaction. Examples of suitable alkylating agents for any one of the embodiments of this invention are olefins, preferably, ethylene, propylene, the butenes, and the pentenes, and mixtures thereof; alcohols (inclusive of monoalcohols, dialcohols, trialcohols, etc.), such as methanol, ethanol, the propanols, the butanols, and the pentanols; aldehydes such as formaldehyde, acetaldehyde, propionaldehyde, butyraldehyde, and n-valeraldehyde; and alkyl halides such as methyl chloride, ethyl chloride, the propyl chlorides, the butyl chlorides, and the pentyl chlorides, and so forth.

Mixtures of light olefins are especially useful as alkylating agents in the alkylation process of this invention. Accordingly, mixtures of ethylene, propylene, butenes, and/or pentenes which are major constituents of a variety of refinery streams, e.g., fuel gas, gas plant off-gas containing ethylene, propylene, etc., naphtha cracker off-gas containing light olefins, refinery FCC propane/propylene streams, etc., are useful alkylating agents herein.

Poly-alkylated aromatic compounds suitable for one or more embodiments of this invention include, but are not limited to, di-ethylbenzenes, tri-ethylbenzenes and poly-ethylbenzene(s), as well as di-isopropylbenzenes (DIPBs), tri-isopropylbenzenes (TIPBs) and poly-isopropylbenzene(s) or mixtures thereof.

### Guard Bed

Generally, the alkylatable aromatic compound stream and the alkylating agent stream supplied to the present process are impure streams and will contain some level of reactive impurities (as defined above), such as, for example, nitrogen compounds, which are small enough to enter the pores of the catalyst, preferably alkylation catalyst and/or transalkylation catalyst, and thereby poison the catalyst. Moreover, it is normal to supply all alkylatable aromatic compounds to the first alkylation and/or transalkylation reaction zone, but to divide and supply the alkylating agent between the alkylation and/or transalkylation catalyst beds. Thus, the catalyst in the first reaction zone is more likely to be poisoned by impurities. Thus, to reduce the frequency with which the catalyst in the first reaction zone must be removed for replacement, regeneration or reactivation, the present process preferably employs a separate guard bed in the first alkylation and/or transalkylation reaction zone. Alternatively, the guard bed may be upstream of and separate from the first reaction zone. The effluent from the guard bed is a treated feed, such as, for example, a treated alkylatable aromatic compound and/or a treated alkylating agent, which is then fed to the process of this invention.

The process of the invention, in one or more embodiments, further comprises the step of contacting said alkylatable aromatic compound and/or said alkylating agent with a treatment material to remove at least a portion of any impurities from said alkylatable aromatic compound or said alkylating agent. The treatment material may be selected from the group consisting of clay, resin, activated alumina, a molecular sieve and combinations thereof. The molecular sieve may be selected from the group consisting Linde X, Linde A, zeolite beta, faujasite, zeolite Y, Ultrastable Y (USY), Dealuminized Y (Deal Y), Rare Earth Y (REY), Ultrahydrophobic Y (UHP-Y), mordenite, TEA-mordenite, ZSM-3, ZSM-4, ZSM-14, ZSM-18, ZSM-20 and combinations thereof.

### Transalkylation Catalyst Composition

In another aspect, the present invention is a transalkylation catalyst which comprises a zeolite having a framework structure selected from the group consisting of FAU, BEA^{∗}, MOR, MWW and mixtures thereof. The zeolite has a silica-alumina molar ratio in a range of 10 to 15. The transalkylation catalyst composition has an external surface area/volume ratio in the range of 40 cm⁻¹ to 85 cm⁻¹, or 40 cm⁻¹ to 80 cm⁻¹, or 45 cm⁻¹ or 75 cm⁻¹.

The zeolite having a FAU framework type may be selected from the group consisting of 13X, Ultrastable Y (USY) and its low sodium variant, dealuminized Y (Deal Y), Ultrahydrophobic Y (UHP-Y), rare earth exchanged Y (REY), rare earth exchanged USY (RE-USY) and mixtures thereof. Preferably, the zeolite having a FAU framework type is USY.

The zeolite having a MOR framework type may be selected from the group consisting of mordenite, EMM-34, TEA-mordenite and mixtures thereof. Preferably, the zeolite having a BEA^{∗} framework type is EMM-34, which is disclosed and described in U.S. Pub. 2016-0221832.

Preferably, the zeolite having a BEA^{∗} framework type is zeolite beta.

The zeolite having a MWW framework type is a MCM-22 family material. Such MCM-22 family material may be selected from the group consisting of MCM-22, PSH-3, SSZ-25, MCM-36, MCM-49, MCM-56, ERB-1, EMM-10, EMM-10-P, EMM-12, EMM-13, UZM-8, UZM-8HS, ITQ-1, ITQ-2, ITQ-30 and mixtures of two or more thereof. Preferably, the MCM-22 family material of the zeolite having MWW framework type is MCM-22 or MCM-49.

In one or more embodiments, the transalkylation catalyst composition is acidic in active form and has protons. The zeolite can be combined in a conventional manner with an oxide binder, such as alumina or silica, such that the final transalkylation contains between 1 and 100 wt.% of the zeolite, based on the weight of the catalyst composition. Alternatively, the acidic transalkylation catalyst composition comprises greater than 0 wt.% up to 99 wt.% of a binder, based on the weight of said transalkylation catalyst composition. The zeolite comprise from 1 wt.% up to 100 wt.%, or from 10 wt.% to 90 wt.%, or from 20 wt.% to 80 wt.% of the transalkylation catalyst composition. Preferably, the zeolite comprises from 65 wt.% to 80 wt.% of said transalkylation catalyst composition.

The binder may be a metal or a mixed metal oxide. The binder may be selected from the group consisting of alumina, silica, titania, zirconia, tungsten oxide, ceria, niobia and combinations thereof.

In a preferred embodiment, the transalkylation catalyst composition of this invention comprises a zeolite having a framework structure selected from the group consisting of FAU, BEA^{∗}, MOR, MWW and mixtures thereof, wherein the silica-alumina molar ratio of said zeolite is in a range of 10 to 15, or in the range 11 to 14, or in the range of 12 to 13:
wherein said FAU framework structure is selected from the group consisting of 13X, low sodium ultrastable Y (USY), dealuminized Y (Deal Y), ultrahydrophobic Y (UHP-Y), rare earth exchanged Y (REY), rare earth exchanged USY (RE-USY), and mixtures thereof,
wherein said zeolite which has said BEA^{∗} framework structure is zeolite beta,
   wherein said zeolite which has MOR framework structure is selected from the group consisting of mordenite, EMM-34, TEA-mordenite, and mixtures thereof;
   wherein said zeolite which has said MWW framework structure is a MCM-22 family material, said MCM-22 family molecular sieve is any one of MCM-22, PSH-3, SSZ-25, MCM-36, MCM-49, MCM-56, ERB-1, EMM-10, EMM-10-P, EMM-12, EMM-13, UZM-8, UZM-8HS, UZM-37, ITQ-1, ITQ-2, ITQ-30, or combinations of two or more thereof;
   wherein said transalkylation catalyst has an external surface area/volume ratio in the range of 40 cm⁻¹ to 85 cm⁻¹; and
   wherein said zeolite comprises from 65 wt.% to 80 wt.% of said transalkylation catalyst composition.

The catalytic activity of the transalkylation catalyst composition of this invention is higher than the catalytic activity of a lower-activity (i.e., higher molar silica-content) transalkylation catalyst composition which comprises said zeolite and has a silica-alumina (Si/Ah) molar ratio in the range of 25 to 37, or in the range 27 to 35, or in the range 29 to 33, when the catalysts are compared under equivalent transalkylation conditions. The higher catalytic activity of the transalkylation catalyst of this invention is achieved by decreasing the amount of silica in the composition which results in a lower silica-alumina molar ratio.

Accordingly, the higher-activity (i.e., lower silica-content) transalkylation catalyst composition of this invention when employed in a process for producing ethylbenzene or cumene, exhibits a weight hourly space velocity of the poly-alkylated benzene stream that is higher than the weight hourly space velocity of a lower-activity (i.e., higher silica-content) transalkylation catalyst composition employed in such process, where the catalysts are compared under equivalent transalkylation conditions, such as equivalent transalkylation temperatures.

Alternatively, the higher-activity (i.e., lower silica-content) transalkylation catalyst composition of this invention when employed in a process for producing ethylbenzene or cumene, may be operated at a lower transalkylation temperature than that of a lower-activity (i.e., higher silica-content) transalkylation catalyst composition employed in such process, where the catalysts are compared under equivalent transalkylation conditions.

Not to be bound by any theory, it is believed that the higher activity exhibited by the transalkylation catalyst composition of this invention is provided by the lower silica-alumina (Si/Ah) molar ratio of the zeolite combined with the higher external surface area/volume (SA/V) of the composition. The lower Si/Al₂ molar ratio provides higher alumina content which facilitates the transalkylation reaction. The higher SA/V ratio provides an increased surface area per unit volume for the transalkylation of the bulky reactants. This is particularly true of a reaction in the liquid phase.

### Alkylation Catalyst

In one or more embodiments, the alkylation catalyst comprises an aluminosilicate. The aluminosilicate is any one of a MCM-22 family molecular sieve, faujasite, mordenite, zeolite-beta, or combinations of two or more thereof. The MCM-22 family molecular sieve is any one of MCM-22, PSH-3, SSZ-25, MCM-36, MCM-49, MCM-56, ERB-1, EMM-10, EMM-10-P, EMM-12, EMM-13, UZM-8, UZM-8HS, UZM-37, ITQ-1, ITQ-2, ITQ-30, or combinations of two or more thereof.

In one or more embodiments, the alkylation catalyst is acidic in active form and has protons. The zeolite can be combined in a conventional manner with an oxide binder, such as alumina or silica, such that the final alkylation catalyst composition contains between 1 and 100 wt.% of the zeolite, based on the weight of said alkylation catalyst composition. Alternatively, the acidic alkylation catalyst composition comprises greater than 0 wt.% up to 99 wt.% of a binder, based on the weight of said alkylation catalyst composition. The zeolite comprise from 1 wt.% up to 100 wt.%, or from 10 wt.% to 90 wt.%, or from 20 wt.% to 80 wt.% of the alkylation catalyst composition. Preferably, the zeolite comprises from 65 wt.% to 80 wt.% of said alkylation catalyst composition.

The binder may be a metal or a mixed metal oxide. The binder may be selected from the group consisting of alumina, silica, titania, zirconia, tungsten oxide, ceria, niobia and combinations thereof.

In one or more embodiments, said alkylation or transalkylation catalyst composition can be a fresh alkylation or transalkylation catalyst composition, an at least partially deactivated alkylation or transalkylation catalyst composition, or combinations thereof. In one or more embodiments, said at least partially deactivated alkylation or transalkylation catalyst was deactivated by coke deposition during its prior use in an alkylation or transalkylation process.

### Catalyst Regeneration

As the alkylation and/or transalkylation process of the invention proceeds, the alkylation and/or transalkylation catalyst composition will gradually lose its alkylation activity, such that the reaction temperature required to achieve a given performance parameter, such as, for example, conversion of the alkylating agent, will increase. When the alkylation and/or transalkylation catalyst activity has decreased by some predetermined amount, typically 5% to 90% and, more preferably 10% to 50%, compared to the initial alkylation and/or transalkylation catalyst activity, the deactivated catalyst composition can be subjected to a regeneration procedure using any known method, such as the method disclosed in U.S. Patent No. 6,380,119 to BASF.

### Examples

The invention will now be more particularly described with reference to the following Examples.

### Effect of USY zeolite Si/Al₂ molar ratio on Transalkylation Performance

### Examples 1 and 2 Catalyst Preparations

In Example 1 (comparative), the catalyst composition (Catalyst A) contained 80 wt.% USY zeolite (Si/Al₂ = 30 molar) and 20 wt.% amorphous Al₂O₃ (alumina) in acidic form.

In Example 2, the catalyst composition (Catalyst B) contained 80 wt.% USY zeolite (Si/Al₂ = 12 molar) and 20 wt.% amorphous Al₂O₃ (alumina) in acidic form.

### Catalyst Transalkylation Performance Evaluation for Examples 1 and 2

Following catalyst preparation, the transalkylation of poly-ethylbenzenes (PEB) with benzene was performed in a fixed bed reactor with Catalyst A and Catalyst B. The PEB stream included di-ethylbenzene (DEB). The test procedure consisted of loading the dried catalyst into a batch reactor along with benzene. The reactor was then heated to 266°F (130°C) followed by the addition of PEB under an inert gas pressure of 300 psig (2068.43 kPa). For Catalyst A, the benzene/PEB ratio of 2:1 by weight and the weight hourly space velocity (WHSV) of 1.1 hr⁻¹ were set, and the reaction temperature increased stepwise to 190°C to achieve a target DEB conversion of 65%. For Catalyst B, the benzene/PEB ratio of 2:1 by weight and the weight hourly space velocity (WHSV) of 1.1 hr⁻¹ were set, and the reaction temperature decreased stepwise to 177°C to achieve a target DEB conversion of 65%. Samples were removed periodically for the duration of the test and analyzed with gas chromatography to determine the conversion of DEB.

Table 1 shows the performance data of Catalyst A and Catalyst B the transalkylation of poly-ethylbenzenes (PEB) with benzene as follows:

**Table 1**

| | Catalyst A (comp.) | Catalyst B | Catalyst A (comp.) | Catalyst B |
|---|---|---|---|---|
| PEB WHSV, (hr⁻¹) | 1.1 | 1.1 | 0.86 | 1.74 |
| Temp, °C | 190 | 177 | 190 | 190 |
| DEB Conv. | 65% | 65% | 65% | 65% |

As can be seen from Table 1, lower temperature operation is achieved at constant DEB conversion and at constant PEB throughput. Alternatively, higher PEB throughput is achieved at constant temperature and constant DEB conversion.

### Effect of USY zeolite Catalyst Particle Size And Shape On Transalkylation Catalyst Activity Examples 3 to 6 Catalyst Preparations

In Example 3 (comparative), the catalyst composition (Catalyst C) contained 80 wt.% USY zeolite (Si/Al₂ = 12 molar) and 20 wt.% of an amorphous Al₂O₃ (alumina) in acidic form and having a particle size and shape of a 1/16 inch cylindrical extrudate.

In Example 4, the catalyst composition (Catalyst D) contained 80 wt.% USY zeolite (Si/Al₂ = 12 molar) and 20 wt.% of an amorphous Al₂O₃ (alumina) in acidic form and having a particle size and shape of a 1/20 inch quadrulobe extrudate.

In Example 5 (comparative), the catalyst composition (Catalyst E) contained 80 wt.% USY zeolite (Si/Al₂ = 12 molar) and 20 wt.% of an amorphous Al₂O₃ (alumina) in acidic form and having a particle size and shape of a 1/16 inch cylindrical extrudate.

In Example 6, the catalyst composition (Catalyst F) contained 80 wt.% USY zeolite (Si/Al₂ = 12 molar) and 20 wt.% of an amorphous Al₂O₃ (alumina) in acidic form and having a particle size and shape of a 1/20 inch quadrulobe extrudate.

While the calculation of cylinder geometry is well known, the calculation of the quadrulobe geometry is more complex. The equations below detailed the calculations involved in determining the surface/volume (SA/V) of the quadrulobe materials.

| Perimeter (P) | *6*π*r* |
|---|---|
| Cross Sectional Area (AX) | (*16*+π)*r²* |
| Particle Surface Area (SAᵢ) | *SAᵢ* = *2Aₓᵢ* + *PᵢL* |
| Volume (Vi) | Vi = *AₓᵢL* |
| SA/V | SA/V = ΣSAᵢ/ΣVᵢ |

Surface Area/Volume ratios were calculated for the quadrulobe and cylinder extrudates by approximating the particles as cylinders with a 0.25 mm diameter and 0.25 mm length. The following table below listed SA/V ratios for the particle sizes tested.

| Name | 1/20 inch Quadrulobe | 1/16 inch Cylinder |
|---|---|---|
| Geometry | Quadrulobe | Cylinder |
| Diameter | 1/20 inch^{∗} | 1/16 inch |
| Length | ¼ inch | ¼ inch |
| SA/V (in⁻¹) | 198 | 88 |
| SA/V (cm⁻¹) | 78 | 35 |

| | | |
|---|---|---|
| ^{∗}Diameter was measured across the minimum. | | |

### Catalyst Activity Evaluation for Examples 3 to 6

Following catalyst preparation, the transalkylation of poly-ethylbenzenes (PEB) with benzene was performed in a fixed bed reactor with Catalyst C and Catalyst D and for Catalyst E and Catalyst F. The test procedure used was the same as described above. The Transalkylation activity was based on the DEB conversion. The transalkylation activity for Catalyst D was normalized to the transalkylation activity for Catalyst C. The transalkylation activity for Catalyst F was normalized to the transalkylation activity for Catalyst WE.

**Table 2**

| Catalyst | Alumina Type | Extrudate Particle Size/Shape | Normalized Transalkylation Activity |
|---|---|---|---|
| Catalyst C (comp.) | Type 1 | 1/16 inch cylindrical | 1 |
| Catalyst D | Type 1 | 1/20 inch quadrulobe | 1.5 |
| Catalyst E (comp.) | Type 2 | 1/16 inch cylindrical | 0.95 |
| Catalyst F | Type 2 | 1/20 inch quadrulobe | 1.3 |

As can be seen, Table 2 shows that the modification of the catalyst extrudate particle size and shape can influence significantly the transalkylation activity. Catalyst particles with smaller diameters and larger surface area to volume ratios are preferred for liquid phase reactions where mass transport limitations may persist.

Certain embodiments and features have been described using a set of numerical upper limits and a set of numerical lower limits. It should be appreciated that ranges from any lower limit to any upper limit are contemplated unless otherwise indicated. Certain lower limits, upper limits and ranges appear in one or more claims below. All numerical values take into account experimental error and variations that would be expected by a person having ordinary skill in the art.

The term "comprising" (and its grammatical variations) as used herein is used in the inclusive sense of "having" or "including" and not in the exclusive sense of "consisting only of' or "consisting of." The terms "a" and "the" as used herein are understood to encompass the plural as well as the singular.

Various terms have been defined above. To the extent a term used in a claim is not defined above, it should be given the broadest definition persons in the pertinent art have given that term as reflected in at least one printed publication or issued patent.

The foregoing description of the disclosure illustrates and describes the present disclosure. Additionally, the disclosure shows and describes only the preferred embodiments but, as mentioned above, it is to be understood that the disclosure is capable of use in various other combinations, modifications, and environments and is capable of changes or modifications within the scope of the concept as expressed herein, commensurate with the above teachings and/or the skill or knowledge of the relevant art.

## Claims

1. A process for producing ethylbenzene or cumene comprising the steps of:
(a) providing an transalkylation catalyst composition to a reaction zone, said transalkylation catalyst comprising a zeolite having a framework structure selected from the group consisting of FAU, BEA^{∗}, MOR, MWW and mixtures thereof, wherein the silica-alumina molar ratio of said zeolite is in a range of 10 to 15, wherein said transalkylation catalyst composition is in the form of an extrudate having an external surface area/volume ratio in the range of 40 cm⁻¹ to 85 cm⁻¹;
(b) providing a stream comprising a poly-alkylated benzene and a portion of an alkylatable aromatic compound stream to said reaction zone, wherein said poly-alkylated benzene stream comprises di-ethylbenzene or di-isopropylbenzene and said alkylatable aromatic compound stream comprises benzene;
(c) contacting said poly-alkylated benzene stream with said alkylatable aromatic compound stream in the presence of said transalkylation catalyst composition under at least partial liquid phase transalkylation conditions to alkylate the alkylatable aromatic compound and produce a transalkylation effluent stream comprising ethylbenzene or cumene;
(d) providing a stream comprising an alkylating agent and another portion of said alkylatable aromatic compound stream to another reaction zone;
(e) contacting said another portion of said alkylatable aromatic compound stream with said stream comprising an alkylating agent under alkylation conditions in the presence of an alkylation catalyst to alkylate the alkylatable aromatic compound and produce an alkylation effluent stream which comprises mono-alkylated benzene and said poly-alkylated benzene;
(f) separating said alkylation effluent stream to recover said poly-alkylated benzene stream; and
(g) supplying at least a portion of said poly-alkylated benzene stream to step (b).

2. The process of claim 1, wherein said alkylatable aromatic compound stream comprising benzene is an impure stream which further comprises nitrogenous impurities.

3. The process of claim 2, further comprising the step of contacting said impure stream with a treatment material under treatment conditions to remove at least a portion of said nitrogenous impurities,
wherein said treatment conditions include a temperature from 30°C to 200°C, a weight hourly space velocity (WHSV) of from 0.1 hr⁻¹ and 200 hr⁻¹, and a pressure between ambient and 3000 kPa,
wherein said treatment material is selected from the group consisting of a clay, a resin, an activated alumina, a molecular sieve and combinations thereof, wherein said molecular sieve is selected from the group consisting of Linde X, Linde A, zeolite beta, faujasite, zeolite Y, Ultrastable Y (USY), Dealuminized Y (Deal Y), Rare Earth Y (REY), Ultrahydrophobic Y (UHP-Y), mordenite, TEA-mordenite, ZSM-3, ZSM-4, ZSM-14, ZSM-18, ZSM-20 and combinations thereof.

4. The process of any preceding claim, further comprising the step:
(h) separating said transalkylation effluent stream to recover an ethylbenzene stream or a cumene stream; and
(i) separating said alkylation effluent stream to recover an ethylbenzene stream or a cumene stream.

5. The process of any preceding claim, wherein said mono-alkylated benzene is ethylbenzene, said poly-alkylated benzene is poly-ethylbenzene and said alkylating agent is ethylene.

6. The process of any preceding claim, wherein said mono-alkylated benzene is cumene, said poly-alkylated benzene is poly-isopropylbenzene, and said alkylating agent is propylene.

7. The process of any preceding claim, wherein said at least partial liquid phase transalkylation conditions include a temperature of 100°C to 300°C, a pressure of 200 kPa to 6000 kPa, weight hourly space velocity (WHSV) based on the total feed of 0.5 hr⁻¹ to 100 hr⁻¹, and an aromatic/poly-alkylated compound weight ratio 1:1 to 6:1; preferably a temperature of 100°C to 200°C and a pressure of 200 kPa to 600 kPa.

8. The process of any preceding claim, wherein said alkylation conditions are at least partially liquid phase conditions and include a temperature of 150°C to 300°C and a pressure of up to 20000 kPa, and a WHSV based on the weight of said alkylating agent from 0.1 hr⁻¹ to 30 hr⁻¹.

9. The process of any preceding claim, wherein said zeolite which has said FAU framework structure is selected from the group consisting of 13X, low sodium ultrastable Y (USY), dealuminized Y (Deal Y), ultrahydrophobic Y (UHP-Y), rare earth exchanged Y (REY), rare earth exchanged USY (RE-USY), and mixtures thereof.

10. The process of any preceding claim, wherein said zeolite which has said BEA^{∗} framework structure is zeolite beta.

11. The process of any preceding claim, wherein said zeolite which has said MOR framework structure is selected from the group consisting of mordenite, EMM-34, TEA-mordenite, and mixtures thereof.

12. The process of any preceding claim, wherein said zeolite which has said MWW framework structure is a MCM-22 family molecular sieve and combinations thereof.

13. The process of claim 12, wherein said MCM-22 family molecular sieve is any one of MCM-22, PSH-3, SSZ-25, MCM-36, MCM-49, MCM-56, ERB-1, EMM-10, EMM-10-P, EMM-12, EMM-13, UZM-8, UZM-8HS, UZM-37, ITQ-1, ITQ-2, ITQ-30, or combinations of two or more thereof.

14. The process of any preceding claim, wherein said alkylation catalyst comprises an aluminosilicate.

15. The process of claim 14, wherein said aluminosilicate is any one of a MCM-22 family molecular sieve, faujasite, mordenite, zeolite-beta, or combinations of two or more thereof.

## Patentansprüche

1. Verfahren zur Herstellung von Ethylbenzol oder Cumol, bei dem in Stufen
(a) einer Reaktionszone eine Transalkylierungskatalysatorzusammensetzung bereitgestellt wird, wobei der Transalkylierungskatalysator Zeolith mit einer Grundgerüststruktur ausgewählt aus der Gruppe bestehend aus FAU, BEA*, MOR, MWW und Mischungen davon umfasst, das Molverhältnis von Siliciumdioxid zu Aluminiumoxid von dem Zeolithen in einem Bereich von 10 bis 15 liegt, wobei die Transalkylierungskatalysatorzusammensetzung in Form eines Extrudats mit einem Verhältnis von Außenoberfläche/Volumen im Bereich von 40 cm⁻¹ bis 85 cm⁻¹ liegt,
(b) der Reaktionszone ein Strom bereitgestellt wird, der polyalkyliertes Benzol und einen Anteil eines alkylierbaren aromatischen Verbindungsstroms umfasst, wobei der polyalkylierte Benzolstrom Diethylbenzol oder Diisopropylbenzol umfasst und der alkylierbare aromatische Verbindungsstrom Benzol umfasst,
(c) der polyalkylierte Benzolstrom mit dem alkylierbaren aromatischen Verbindungsstrom in Gegenwart der Transalkylierungskatalysatorzusammensetzung unter mindestens teilweise Flüssigphasen-Transalkylierungsbedingungen in Kontakt gebracht wird, um die alkylierbare aromatische Verbindung zu alkylieren und einen Transalkylierungsausflussstrom zu produzieren, der Ethylbenzol oder Cumol umfasst,
(d) einer anderen Reaktionszone ein Strom bereitgestellt wird, der Alkylierungsmittel und einen anderen Anteil des alkylierbaren aromatischen Verbindungsstroms umfasst,
(e) der andere Anteil des alkylierbaren aromatischen Verbindungsstroms unter Alkylierungsbedingungen in Anwesenheit von Alkylierungskatalysator mit dem Alkylierungsmittel umfassenden Strom in Kontakt gebracht wird, um die alkylierbare aromatische Verbindung zu alkylieren und einen Alkylierungsausflussstrom zu produzieren, der monoalkyliertes Benzol und das polyalkylierte Benzol umfasst,
(f) der Alkylierungsausflussstrom getrennt wird, um den polyalkylierten Benzolstrom zu gewinnen, und
(g) Schritt (b) mindestens ein Anteil des polyalkylierten Benzolstroms zugeführt wird.

2. Verfahren nach Anspruch 1, bei dem der alkylierbare aromatische Verbindungsstrom, der Benzol umfasst, ein unreiner Strom ist, der des Weiteren stickstoffhaltige Verunreinigungen umfasst.

3. Verfahren nach Anspruch 2, bei dem in einem weiteren Schritt der unreine Strom mit Behandlungsmaterial unter Behandlungsbedingungen in Kontakt gebracht wird, um mindestens einen Anteil der stickstoffhaltigen Verunreinigungen zu entfernen,
wobei die Behandlungsbedingen eine Temperatur von 30°C bis 200°C, einen stündlichen Massendurchsatz (WHSV) von 0,1 h⁻¹ bis 200 h⁻¹ und einen Druck zwischen Umgebungsdruck und 3000 kPa einschließen,
wobei das Behandlungsmaterial ausgewählt ist aus der Gruppe bestehend aus Ton, Harz, aktiviertem Aluminiumoxid, Molekularsieb und Kombinationen davon, wobei das Molekularsieb ausgewählt ist aus der Gruppe bestehend aus Linde X, Linde A, Zeolith beta, Faujasit, Zeolith Y, ultrastabilem Y (USY), entaluminiertem Y (Deal Y), Seltenerd-Y (REY), ultrahydrophobem Y (UHP-Y), Mordenit, TEA-Mordenit, ZSM-3, ZSM-4, ZSM-14, ZSM-18, ZSM-20 und Kombinationen davon.

4. Verfahren nach einem der vorhergehenden Ansprüche, das des Weiteren den Schritt umfasst, in dem
(h) der Transalkylierungsausflussstrom getrennt wird, um einen Ethylbenzolstrom oder Cumolstrom zu gewinnen, und
(i) der Alkylierungsausflussstrom getrennt wird, um einen Ethylbenzolstrom oder Cumolstrom zu gewinnen.

5. Verfahren nach einem der vorhergehenden Ansprüche, bei dem das monoalkylierte Benzol Ethylbenzol ist, das polyalkylierte Benzol Polyethylbenzol ist, und das Alkylierungsmittel Ethylen ist.

6. Verfahren nach einem der vorhergehenden Ansprüche, bei dem das monoalkylierte Benzol Cumol ist, das polyalkylierte Benzol Polyisopropylbenzol ist, und das Alkylierungsmittel Propylen ist.

7. Verfahren nach einem der vorhergehenden Ansprüche, bei dem die mindestens teilweisen Flüssigphasen-Transalkylierungsbedingungen eine Temperatur von 100°C bis 300°C, einen Druck von 200 kPa bis 6000 kPa, einen stündlichen Massendurchsatz (WHSV), bezogen auf das Gesamteinsatzmaterial, von 0,5 h⁻¹ bis 100 h⁻¹ sowie ein Gewichtsverhältnis von Aromat/polyalkylierter Verbindung von 1:1 bis 6:1 einschließen, vorzugsweise eine Temperatur von 100°C bis 200°C und einen Druck von 200 kPa bis 600 kPa.

8. Verfahren nach einem der vorhergehenden Ansprüche, bei dem die Alkylierungsbedingungen mindestens teilweise Flüssigphasenbedingungen sind und eine Temperatur von 150°C bis 300°C und einen Druck von bis zu 20000 kPa und ein WHSV, bezogen auf das Gewicht des Alkylierungsmittels, von 0,1 h⁻¹ bis 30 h⁻¹ einschließen.

9. Verfahren nach einem der vorhergehenden Ansprüche, bei dem der Zeolith, der die FAU-Grundgerüststruktur aufweist, ausgewählt ist aus der Gruppe bestehend aus 13X, natriumarmem ultrastabilem Y (USY), entaluminiertem Y (Deal Y), ultrahydrophobem Y (UHP-Y), Seltenerd-ausgetauschtem Y (REY), Seltenerd-ausgetauschtem USY (RE-USY) und Mischungen davon.

10. Verfahren nach einem der vorhergehenden Ansprüche, bei dem der Zeolith, der die BEA*-Grundgerüststruktur aufweist, Zeolith beta ist.

11. Zeolith nach einem der vorhergehenden Ansprüche, bei dem der Zeolith, der die MOR-Grundgerüststruktur aufweist, ausgewählt ist aus der Gruppe bestehend aus Mordenit, EMM-34, TEA-Mordenit und Mischungen davon.

12. Verfahren nach einem der vorhergehenden Ansprüche, bei dem der Zeolith, der die MWW-Grundgerüststruktur aufweist, Molekularsieb der Familie MCM-22 und Kombinationen davon ist.

13. Verfahren nach Anspruch 12, bei dem das Molekularsieb der Familie MCM-22 ein beliebiges von MCM-22, PSH-3, SSZ-25, MCM-36, MCM-49, MCM-56, ERB-1, EMM-10, EMM-10-P, EMM-12, EMM-13, UZM-8, UZM-8HS, UZM-37, ITQ-1, ITQ-2, ITQ-30 oder Kombinationen von zwei oder mehr davon ist.

14. Verfahren nach einem der vorhergehenden Ansprüche, bei dem der Alkylierungskatalysator Aluminiumsilikat umfasst.

15. Verfahren nach Anspruch 14, bei dem das Aluminiumsilikat ein beliebiges von Molekularsieb der Familie MCM-22, Faujasit, Mordenit, Zeolith beta oder Kombinationen von zwei oder mehr davon ist.

## Revendications

1. Procédé pour la production d'éthylbenzène ou de cumène comprenant les étapes de :
(a) fourniture d'une composition de catalyseur de transalkylation à une zone de réaction, ledit catalyseur de transalkylation comprenant une zéolithe possédant une structure de cadre choisie dans le groupe constitué par FAU, BEA*, MOR, MWW et des mélanges correspondants, le rapport molaire de silice-alumine de ladite zéolithe étant dans une plage de 10 à 15, ladite composition de catalyseur de transalkylation étant sous la forme d'un extrudat possédant un rapport de superficie externe/volume dans la plage de 40 cm⁻¹ à 85 cm⁻¹ ;
(b) fourniture d'un flux comprenant un benzène poly-alkylé et une partie d'un flux de composé aromatique pouvant être alkylé à ladite zone de réaction, ledit flux de benzène poly-alkylé comprenant du di-éthylbenzène ou du di-isopropylbenzène et ledit flux de composé aromatique pouvant être alkylé comprenant du benzène ;
(c) mise en contact dudit flux de benzène poly-alkylé avec ledit flux de composé aromatique pouvant être alkylé en la présence de ladite composition de catalyseur de transalkylation dans des conditions de transalkylation à phase liquide au moins partielle pour alkyler le composé aromatique pouvant être alkylé et produire un flux d'effluent de transalkylation comprenant de l'éthylbenzène ou du cumène ;
(d) fourniture d'un flux comprenant un agent d'alkylation et une autre partie dudit flux de composé aromatique pouvant être alkylé à une autre zone de réaction ;
(e) mise en contact de ladite autre partie dudit flux de composé aromatique pouvant être alkylé avec ledit flux comprenant un agent d'alkylation dans des conditions d'alkylation en la présence d'un catalyseur d'alkylation pour alkyler le composé le composé aromatique pouvant être alkylé et produire un flux d'effluent d'alkylation qui comprend un benzène mono-alkylé et ledit benzène poly-alkylé ;
(f) séparation dudit flux d'effluent d'alkylation pour récupérer ledit flux de benzène poly-alkylé ; et
(g) alimentation d'au moins une partie dudit flux de benzène poly-alkylé à l'étape (b).

2. Procédé selon la revendication 1, ledit flux de composé aromatique pouvant être alkylé comprenant du benzène étant un flux impur qui comprend en outre des impuretés azotées.

3. Procédé selon la revendication 2, comprenant en outre l'étape de mise en contact dudit flux impur avec un matériau de traitement dans des conditions de traitement pour éliminer au moins une partie desdites impuretés azotées,
lesdites conditions de traitement comprenant une température de 30 °C à 200 °C, une vitesse spatiale horaire en poids (WHSV) allant de 0,1 h⁻¹ à 200 h⁻¹, et une pression comprise entre la pression ambiante et 3 000 kPa,
ledit matériau de traitement étant choisi dans le groupe constitué par une argile, une résine, une alumine activée, un tamis moléculaire et des combinaisons correspondantes, ledit tamis moléculaire étant choisi dans le groupe constitué par Linde X, Linde A, zéolithe bêta, faujasite, zéolithe Y, Y Ultrastable (USY), Y désaluminisée (Deal Y), Y Terre Rare (REY), Y Ultrahydrophobe (UHP-Y), mordénite, TEA-mordénite, ZSM-3, ZSM-4, ZSM-14, ZSM-18, ZSM-20 et des combinaisons correspondantes.

4. Procédé selon une quelconque revendication précédente, comprenant en outre l'étape :
(h) séparation dudit flux d'effluent de transalkylation pour récupérer un flux d'éthylbenzène ou un flux de cumène ; et
(i) séparation dudit flux d'effluent d'alkylation pour récupérer un flux d'éthylbenzène ou un flux de cumène.

5. Procédé selon une quelconque revendication précédente, ledit benzène mono-alkylé étant l'éthylbenzène, ledit benzène poly-alkylé étant un poly-éthylbenzène et ledit agent d'alkylation étant l'éthylène.

6. Procédé selon une quelconque revendication précédente, ledit benzène mono-alkylé étant le cumène, ledit benzène poly-alkylé étant un poly-isopropylbenzène, et ledit agent d'alkylation étant le propylène.

7. Procédé selon une quelconque revendication précédente, lesdites conditions de transalkylation à phase liquide au moins partielle comprenant une température de 100 °C à 300 °C, une pression de 200 kPa à 6 000 kPa, une vitesse spatiale horaire en poids (WHSV) basée sur la charge d'alimentation totale allant de 0,5 h⁻¹ à 100 h⁻¹, et un rapport en poids de composé aromatique/poly-alkylé de 1 : 1 à 6 : 1 ; préférablement une température de 100 °C à 200 °C et une pression de 200 kPa à 600 kPa.

8. Procédé selon une quelconque revendication précédente, lesdites conditions d'alkylation étant des conditions à phase au moins partiellement liquide et comprenant une température de 150 °C à 300 °C et une pression allant jusqu'à 20 000 kPa, et une WHSV basée sur le poids dudit agent d'alkylation de 0,1 h⁻¹ à 30 h⁻¹.

9. Procédé selon une quelconque revendication précédente, ladite zéolithe qui possède ladite structure de cadre FAU étant choisie dans le groupe constitué par 13X, Y ultrastable à faible teneur en sodium (USY), Y désaluminisée (Deal Y), Y ultrahydrophobe (UHP-Y), Y échangée avec une terre rare (REY), USY échangée avec une terre rare (RE-USY), et des mélanges correspondants.

10. Procédé selon une quelconque revendication précédente, ladite zéolithe qui possède ladite structure de cadre BEA* étant une zéolithe bêta.

11. Procédé selon une quelconque revendication précédente, ladite zéolithe qui possède ladite structure de cadre MOR étant choisie dans le groupe constitué par mordénite, EMM-34, TEA-mordénite, et des mélanges correspondants.

12. Procédé selon une quelconque revendication précédente, ladite zéolithe qui possède ladite structure de cadre MWW étant un tamis moléculaire de la famille du MCM-22 et des combinaisons correspondantes.

13. Procédé selon la revendication 12, ledit tamis moléculaire de la famille du MCM-22 étant l'un quelconque parmi MCM-22, PSH-3, SSZ-25, MCM-36, MCM-49, MCM-56, ERB-1, EMM-10, EMM-10-P, EMM-12, EMM-13, UZM-8, UZM-8HS, UZM-37, ITQ-1, ITQ-2, ITQ-30, et des combinaisons de deux ou plus de ceux-ci.

14. Procédé selon une quelconque revendication précédente, ledit catalyseur d'alkylation comprenant un aluminosilicate.

15. Procédé selon la revendication 14, ledit aluminosilicate étant l'un quelconque parmi un tamis moléculaire de la famille du MCM-22, faujasite, mordénite, zéolithe-bêta, et des combinaisons de deux ou plus de ceux-ci.
